# EUROPEAN PATENT APPLICATION

(11) **EP 4 745 572 A1**
(43) Date of publication of application: **20.05.2026**
(21) Application number: 24213992.1
(22) Date of filing: 19.11.2024
(51) Int. Cl.: G01N 33/543, B01L 3/00, G01N 27/327

(54) **DEVICE FOR AUTONOMOUS MEASUREMENT IN COMPLEX SAMPLES AND FOR THE AUTONOMOUS EXTRACTION OF A LIQUID SAMPLE**

(71) Applicant: Instituto Tecnológico de la Energía, 46980 Paterna Valencia (ES); Roxhed, Niclas, 167 33 Bromma (SE); Stemme, Göran, 181 30 Lidingö (SE); Ribet, Federico, 11251 Stokholm (SE); Hedberg, Ellinor, 18769 Täby (SE)
(72) Inventor: Roxhed, Niclas, 16733 Bromma (SE); Stemme, Göran, 18130 Lidingö (SE); Ribet, Federico, 11251 Stokholm (SE); Hedberg, Ellinor, 18769 Täby (SE); García Carmona, Laura, Valencia (ES); Lucas Garrote, Beatriz, Valencia (ES); Vegas García, Marta, Valencia (ES); García Pellicer, Marta, Valencia (ES); Quijano López, Alfredo, Valencia (ES)
(74) Representative: Lozano Alonso, Jose

(57) **Abstract**

Lateral flow strip for the autonomous realization of chemical and/or biological reactions in a complex liquid sample comprising at least one porous hydrophilic layer, with at least one zone of placement of the liquid sample, where the lateral flow is configured to transport the fluid along the porous hydrophilic layer, characterized in that it includes means of regulating the flow velocity of fluid along the lateral flow strip, configured to regulate the reaction time of the liquid sample in the different stages of reaction. Biosensor comprising the lateral flow strip and an electrode located in direct contact with the lateral flow strip, configured to interact with the target molecule of the measurement. Device comprising the lateral flow strip, the electrode and an autonomous capillarity extraction element, where the capillarity effect of the extraction element is greater than the capillarity effect of the tissue from which the liquid sample is extracted.

## Description

### TECHNICAL FIELD

The present invention refers to bioassays, biosensors and devices for conducting biological tests in general.

In particular, the present invention refers to a device that comprises a combination of an electrochemical sensor with a lateral flow strip, for the realization of qualitative, quantitative or semiquantitative measurements, sample treatment, and specific recognition reactions autonomously.

In addition, the present invention also refers to the previous device that includes, in addition, an autonomous system of extraction of liquid samples to be analyzed by the electrochemical sensor.

### BACKGROUND OF THE INVENTION

Lateral flow assays or LFAs are easy-to-use screening devices that are used to quickly confirm the presence or absence of a target molecule, such as proteins in samples of human specimens, toxins in feed or antibiotics in dairy products.

Lateral flow immunoassays (IFL) are based on the use of nitrocellulose membranes as a support for immune reactions. They are tests used to detect the presence or absence of a molecule of analytical interest (analyte of interest) in a liquid sample.

Unlike traditional immunoassays that involve the use of multi-well plates, a high cost, complicated washing and sample preparation procedures, long detection times, etc., lateral flow immunoassays are portable, fast and easy to use, which allows the analysis to be carried out in a delocalized way and by personnel without scientific qualification. Its use is widespread in the human health sector. Although currently, given its versatility, its participation is increasingly important in the pharmaceutical, environmental and agri-food sector among others.

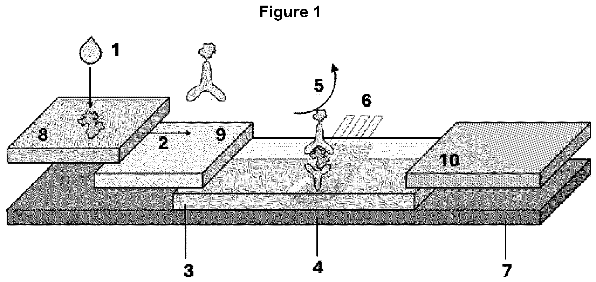

The lateral flow tests 14, as shown in Figure 1, are made up of a series of membranes 3 of porous material that are superimposed on each other and attached to a solid support 7, or a support card or backing card.

The test begins with the addition of sample 1 to the sample pad 8 where it is treated to be compatible with the rest of the test. The treated sample migrates from sample pad 8, to the conjugate pad 9 that contains the immobilized conjugate. Generally, the conjugation is done with colloidal gold particles, although it can also be done with colored, paramagnetic or fluorescent latex particles, among others.

The sample re-mobilizes the conjugate and interacts with the analyte when both migrate to the next section of the strip, which is the capture zone 4. In capture zone 4, other biological components have been immobilized that have been deposited in specific lines of the membrane where they serve to capture the analyte and conjugate as they migrate through the test line(s).

In this type of test, we will be able to detect it visually with the appearance of a colored band. Likewise, the migration also usually involves control molecules that in the capture zone 4 are transformed into a colored band on the control line (control line) that indicates that the test is valid. This allows non-specialized users to detect visual and qualitative measurements.

The excess of reagents passes through the capture area until it reaches the absorbent pad 10.

Among the best-known LF tests are pregnancy tests or, currently, the rapid test for the detection of antibodies against SARS-CoV-2.

However, this type of test is carried out by visual optical detection, so it is difficult to achieve a quantitative analysis in complex samples since it would require additional equipment not compatible by it use and interpretation by non-specialized users.

### SUMMARY OF THE INVENTION

The device for the measurement and autonomous treatment of molecules in complex samples and/or for the autonomous extraction of a liquid sample is configured, therefore, as a remarkable novelty within its field of application, since, according to its implementation and exhaustively, the objectives indicated below are achieved, being the characterizing details that make it possible and that distinguish them, conveniently included in the final claims that accompany this description.

The present invention presents an autonomous sample processing and measurement device for the automatic performance of electrochemical lateral flow tests to detect any kind of molecule in complex samples such as sap, sweat, saliva, urine, etc., with little or no human intervention.

This system is based on the use of a lateral flow system, coupled to an electrochemical detection, with the peculiarity of having a design and modification specially adapted to the performance of bioassays autonomously, where all the stages of the process could occur automatically, with low or no intervention by users, which allows the realization of complex electrochemical bioassays by non-specialized users.

On the other hand, the device for the autonomous measurement of molecules in complex samples not only allows the performance of bioassays autonomously, but also the taking of samples, minimizing/reducing/drastically eliminating the steps of human intervention and time of the analysis.

To achieve this, the device must be coupled to an extraction element that allow for fluid extraction by capillarity. The element could consist of glass, plastic or any other non-absorbent hydrophilic material where the capillarity effect of the extraction element and the evaporation is superior to the capillarity effect of the tissue from which the sample is extracted. This would allow for a flow of the liquid sample from the analyzed tissue to the measuring device.

As is known, electrochemical lateral flow tests, unlike the classic LF tests, which are optical detection, allow qualitative and quantitative detection, while being compatible with the new trends of the internet of things (IoT, Internet-of-Things), since the electrical signal, unlike in visual exploration, is susceptible to being sent to an external device or to the cloud.

In response to the above, the proposed system is compatible with wireless technology so it can be easily integrated into the IoT trend, representing the first wearable device capable of being adapted to the quantitative monitoring of any complex molecule autonomously (proteins, DNA, RNA, among others), without human intervention and with low sample volume (3-30µL) in times between 5-30 minutes.

The advantage of the present invention with respect to the state of the art is the possibility of configuring a device with any size and shape that, by varying the dimensions of the strips, the different barriers and the combination of different types of strips, along with the specific modification of the measurement electrode, allows the autonomous or semi-autonomous detection of any type of molecule found in biological samples, even those that require prior sample treatment, obtaining quantitative or semiquantitative measurements. In this way, with a small amount applied/extracted sample, any user can obtain the desired measurements in reduced times, even when it is an unqualified user.

Another advantage is the possibility of obtaining a device that not only allows autonomous or semi-autonomous detection, but also allows autonomous extraction, allowing sampling and further detection without user intervention.

The device for the autonomous measurement of molecules in complex samples and/or for the autonomous extraction of a liquid sample, and the set of elements described, represent an innovation of structural and constitutive characteristics unknown until now, reasons that, together with their practical usefulness, provide it with sufficient foundation to obtain the privilege of exclusivity that is requested.

### DESCRIPTION OF THE DRAWINGS

To complement the description that is being made and, in order to help a better understanding of the characteristics of the invention, it is accompanied as an integral part of the said description, a set of drawings in which, for illustrative and not limiting, the following has been represented:
- Figure 1.- Example of a test using lateral flow strips from the state of the art including electrochemical detection 6.
- Figure 2.- Example of different means of regulating the speed of fluid circulation along the lateral flow strip

### List of references:

1. Sample
2. Fluid
3. Membranes
4. Capture zone
5. Electrochemical signal
6. Electrode
7. Solid support
8. Sample pad
9. Conjugate pad
10. Absorbent pad
11. Water-soluble barriers
12. Alternative paths
13. Different types of porous materials
14. Lateral Flow test

### PREFERRED EMBODIMENTS OF THE INVENTION

In the following detailed description of the preferred embodiments, reference is made to the attached drawings that are part of this report, and in which preferred embodiments in which the invention can be carried out are shown as an illustration.

In particular, the present invention starts from a lateral flow strip that is specially configured to regulate the reaction time of a liquid sample, so that tests can be carried out autonomously, only with the introduction of the liquid sample. In some applications, the lateral flow strip is compatible with multiplexing of different parameters by modifying the lateral flow strip itself or the electrode associated with it.

To achieve this, the present invention describes a lateral flow strip for the autonomous realization of chemical/biological reactions in a complex liquid sample. It is comprised of at least one porous hydrophilic layer with variable dimensions and geometries (height/thickness, width and depth as well as different geometries where multiple components can be combined to create the desired configuration) and containing at least one zone of placement of the liquid sample and a capture zone in which the reaction of the liquid sample is carried out with the reagents present in the capture zone. The lateral flow strip is configured to transport the fluid along the hydrophilic layers to the region in contact with the electrode, where the measurement is performed. One embodiment of the lateral flow strip is a porous matrix that can be made from paper, cotton, cellulose fibers, polymers or any other porous material that enables capillarity. It could be for example Sartorius Qualitative Grade 601/N Filter Paper or Ahlstrom grade 222 paper. The design can be a circular component with perpendicular strips that enables movement of the liquid sample to the specific regions. The porous matrix can be designed, and the dimensions specified though computer aided design and/or illustrations that are used as an input for a cutter plotter, laser or similar technique for creating the required flow strip.

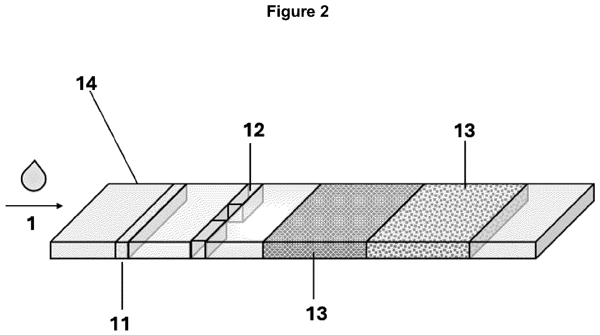

Specifically, the lateral flow strip is characterized by comprising means of regulating the velocity of circulation of the fluid 2 along the lateral flow strip, configured to regulate the reaction time of the liquid sample in the different stages of reaction. Different examples can be seen in Figure 2.

In this way, it is possible to adapt the speed of circulation of the fluid 2 along the lateral flow strip 14, allowing the time of permanence of the fluid 2 in the different stages of reaction in the capture zone 4 and along the strip, to be ideal for the realization of the aforementioned stages of recognition, and reactions, allowing the realization of a plurality of tests autonomously, including immunoassays of several ELISA-type stages, only having to introduce the liquid sample 1.

In a preferred embodiment, the means of regulating the speed of circulation of the fluid are physical, chemical and/or physicochemical barriers.

In a preferred embodiment, the means of regulating the speed of circulation of the fluid are water-soluble barriers 11.

In a preferred embodiment, the means of regulating the flow velocity of the fluid include alternative paths 12 for the flow of the fluid within the lateral flow strip.

In a preferential embodiment, the means of regulating the speed of circulation of the fluid include the use of different types of porous material 13.

In a preferred embodiment, the means of regulating the flow velocity of the fluid include two or more methods of the following: water-soluble barriers 11 and/or alternative paths 12 for the flow of the fluid 2 and/or use of different types of porous material 13.

Any of the above means of regulation, in isolation or in combination of several of them, allows to achieve the objective of regulating the speed of circulation of the fluid in the lateral flow strip, controlling the reaction time in each of the reaction stages in the capture stage.

### 1° OBJECTIVE OF THE INVENTION - AUTONOMOUS BIOSENSE DEVICE

A first objective of the previously defined Lateral Flow Strip, is a biosensor for the autonomous realization of a measurement of an analyte in a liquid and complex sample, without prior treatment, using electrochemical techniques combined with lateral flow strips to control the sequence of the electrochemical reactions involved in the detection and/or quantification of the analyte of interest from the control of the flow of the fluid.

In particular, a biosensor is described for the autonomous measurement of an analyte in a liquid sample, which includes:
- A lateral flow strip 14, formed from a plurality of hydrophilic supports of porous material superimposed, such as paper strips, with at least one zone of placement of the liquid sample and a capture zone 4 in which the chemical/biological reactions of the analyte are carried out with the reagents present in the capture zone 4; configured to transport the fluid 2 along the rest of the porous layers due to their hydrophilic properties.
- And an electrode 6 located in direct contact with the lateral flow strip 14, configured to interact with the target molecule of the measurement.

Where, the biological recognition reaction can occur both in the lateral flow strip 14 and on the surface of the electrode 6.

And where the lateral flow strip 14 comprises means of regulating the flow velocity of the fluid 2 along the lateral flow strip 14, configured to regulate the reaction time of each of the stages of the biological reconnaissance event, necessary to obtain the corresponding signal.

This allows to measure any type of molecule in liquid samples, such as water, sap, blood, urine, saliva or mucosa, as well as in any other type of biological or non-biological fluid; and adapt the biological recognition reaction for the detection of any molecule of interest, such as: ions, proteins, DNA, RNA, etc.

In a preferred embodiment, the means of regulating the speed of circulation of the fluid in the lateral flow strip are physical, chemical and/or physicochemical barriers, such as water-soluble barriers 11 configured to stop the advance of the fluid.

In another preferred embodiment, the means of speed regulation include alternative paths 12 for the flow of the fluid 2 within the lateral flow strip 14.

In another preferred embodiment, the means of regulating the speed of the fluid include the use of different types of porous material 13.

In a preferred embodiment, the biosensor electrode 6 is an electrode made of a biologically and/or chemically modified material, such as carbon, graphene, gold or other screen-printed material. Preferably, the material is modified with a redox/conductor polymer or other redox/conductor molecules.

One of the novelties of the present invention is the possibility of using any electrode 6 of those available on the market, in conjunction with the lateral flow strips 14 claimed for the realization of autonomous measures.

This allows that, by changing the material of the electrode 6, we change the conductivity of the same and the type of subsequent modifications. For example, gold electrodes are more conductive than carbon or graphene electrodes and can be modified in a covalent way, with greater resistance, with molecules that have thiol groups, configured as a simple modification. In the case of graphene, it has a greater conduction compared to carbon, however, non-covalent bonds are usually used for the modification, so it is a greater difficulty. In return, graphene is more sensitive to variations on the surface of the electrode, such as the interaction of the analyte itself.

Therefore, depending on the application, the invention allows the use of the best type of electrode 6 and modification.

In another preferred embodiment, the electrode 6 is a screen-printed carbon electrode (SPCE) and modified with a conductive polymer, such as polyaniline, for the covalent immobilization of the target molecule or the specific receptor that interacts with the target molecule, which can be an antibody, DNA, RNA, enzyme, etc.

In another preferred embodiment, the lateral flow strip 14 contains the anti-target molecule of the specific antibody functionalized with HRP as a redox indicator. The detection of the target of interest is inferred through the electrochemical reaction of the HRP enzyme in the presence of its hydroquinone substrate and hydrogen peroxide by a chronoamperometry measurement for 5 min applying -0.2V. The test results in a higher electrochemical signal when the target concentration in the samples is lower.

In a preferred embodiment, the device comprises a sealing and insulation structure, configured to prevent the evaporation of the sample 1 in the areas of extraction and transport of the fluid to the capture/detection area, so the proposed design can take advantage of and work with small volumes of samples, between 3-30 *µ*l approximately, which are considerably lower than those usually used for the conventional lateral flow tests and allows wearable use of the technology in cases where sample is limited such is the case of sap or interstitial fluid.

This is because the area of the evaporation region located on the lateral flow strip influences the sample flow in the strip and the time that it is being kept in motion. If the entire strip would be exposed to air, evaporation would occur over the entire area and thus affecting the flow characteristics to the favor of the evaporation of the sample. Thereby, a greater volume of sample would be required to cover the entire reaction area.

On the contrary, if it is sealed well and only a small area is exposed, the sample volume can be more efficiently used since evaporation is reduced and the liquid remains in motion for a longer time. Thus, requiring a smaller sample volume to carry out the test.

The novelty of the first invention lies in the use of different wax barriers / different flow path / different dimensions / different types of hydrophilic layers to modulate the flow velocity and, therefore, the reaction time of each of the stages of the bio-recognition event necessary to obtain the specific electrochemical signal for the qualitative or quantitative detection of the analyte of interest with low human intervention.

### 2° OBJECTIVE OF THE INVENTION - AUTONOMOUS SAMPLE COLLECTION DEVICE

It is a second objective of the present invention, to achieve a device that allows the autonomous measurement of analytes in a liquid sample and, in addition, the extraction of liquid samples also autonomously of tissue, such as the stem of a plant or human skin.

Specifically, a device that comprises a biosensor according to the previous realizations is described, that is, it comprises a biosensor for the autonomous realization of the measurement of an analyte in a liquid and complex sample using electrochemical techniques combined with lateral flow strips to control the sequence of the reactions involved in the detection and/or quantification of the analyte of interest from the control of the advance of the fluid, which includes:
- A lateral flow strip 14, formed from a plurality of overlapping porous material membranes, such as paper strips or other porous matrices, with at least one area for placement of the liquid sample and a capture zone in which the chemical reactions of the analyte are carried out with the reagents present in the capture zone; configured to transport the fluid along the plurality of membranes of porous material due to its hydrophilic properties.
- And an electrode 6 located in direct contact with the lateral flow strip, configured to interact with the target molecule of the measurement.

Where, the biological recognition reaction can occur both on the lateral flow strip and on the surface of the electrode.

And where the lateral flow strip 14 comprises means of regulating the flow velocity of the fluid 2 along the lateral flow strip 14, configured to regulate the reaction time of each of the stages of the biological reconnaissance event, necessary to obtain the corresponding signal.

Additionally, the device comprises an autonomous extraction element that allows fluid extraction by capillarity such as glass, plastic or any other non-absorbent hydrophilic material. This can be connected to the capillary matrix/lateral flow strip, where the capillarity effect of the extraction element attached to the matrix combined with evaporation generates a force greater than the capillarity of the tissue from which the sample is extracted.

In this way, due to the difference in the capillarity effect, the circulation of the liquid sample from the analyzed tissue to the biosensor is favored.

Thanks to the combination of the biosensor with the autonomous extraction element, a completely autonomous device is achieved for the performance of in situ analysis of biochemical parameters, both in terms of measurement and sampling and its pretreatment, minimizing/eliminating the steps of human intervention

In a preferential realization, the device comprises a sealing and insulation structure, configured to prevent the evaporation of the sample in the areas of extraction and transport of the fluid to the capture/detection area, so the proposed design can take advantage of and work with small volumes of samples, between 3-30 *µ*l approximately, which are considerably lower than those usually used for the conventional lateral flow tests.

This is because the area of the evaporation region influences the sample in the lateral flow strip by regulating the time it is kept in motion. If the flow strip would be fully exposed to air, evaporation occurs in the entire area of the paper. Thus evaporation of the sample is favored and thereby a greater volume of sample is required to cover the entire reaction area.

On the contrary, if it is sealed well and only a small area of the test strip is exposed to the environment, the sample volume is used more efficiently, remaining longer in motion and thus a smaller sample volume is required to carry out the test.

Sufficiently described the nature of the present invention, as well as the way to put it into practice, it is not considered necessary to make its explanation more extensive so that any expert in the field understands its scope and the advantages that are derived from it, stating that, within its essentiality, it may be put into practice in other forms of realization that differ in detail from the one indicated by way of example, and to which the protection that is obtained will also reach, provided that its fundamental principle is not altered, modified or changed.

## Claims

1. **Lateral flow strip for the autonomous realization of chemical and/or biological reactions in a complex liquid sample** comprising at least one porous hydrophilic layer, with at least one zone of placement of the liquid sample, where the lateral flow strip (14) is configured to transport the fluid (2) along the porous hydrophilic layer, **characterized in that** it includes means of regulating the flow velocity of fluid (2) along the lateral flow strip (14), configured to regulate the reaction time of the liquid sample in the different stages of reaction.

2. **Lateral flow strip** according to claim 1 **characterized by** comprising a capture zone (4) for the treatment of the sample, in which the chemical and/or biological reactions of the liquid sample are carried out, with the reagents present in the capture zone.

3. **Lateral flow strip** according to claims 1 and 2 **characterized in that** the means of regulating the flow velocity of the fluid (2) are physical, chemical and/or physicochemical barriers.

4. **Lateral flow strip** according to the previous claim **characterized in that** the means of regulating the flow velocity of the fluid (2) are water-soluble barriers (11).

5. **Lateral flow strip** according to claims 1 and 2 **characterized in that** the means of regulating the flow velocity of the fluid (2) comprise alternative paths (12) for the flow of the fluid within the lateral flow strip.

6. **Lateral flow strip** according to claims 1 and 2 **characterized in that** the means of regulating the flow velocity of the fluid (2) include the use of different membranes of porous material (13).

7. **Lateral flow strip** according to claims 1 and 2 **characterized in that** the means of regulating the flow velocity of the fluid include two or more of the following methods: water-soluble barriers (11) and/or alternative paths (12) for the flow of the fluid and/or use of different membranes of porous material (13).

8. **Biosensor for the autonomous realization of a measurement of any analyte in a complex liquid sample,** which includes at least:
• a lateral flow strip (14) comprised of one or more membranes (3) of porous material, with at least one area of placement of the liquid sample, where the lateral flow strip (14) is hydrophilic and is configured to transport the fluid along the porous material membranes (3);
• and an electrode (6) located in direct contact with the lateral flow strip (14), configured to interact with the target molecule of the measurement. **characterized in that** the biological recognition reaction occurs in the lateral flow strip (14) and/or on the surface of the electrode (6);
and **in that** the lateral flow strip (14) comprises means of regulating the speed of circulation of the fluid along the lateral flow strip (14), configured to regulate the reaction time of each of the stages of chemical and/or biological reactions.

9. **Biosensor** according to claim 8 **characterized by** comprising a capture area for the treatment of the sample, in which the chemical and/or biological reactions of the liquid sample are carried out, with the reagents present in the capture area (4).

10. **Biosensor** according to the previous claims **characterized in that** the means of regulating the speed of circulation of the fluid are physical, chemical and/or physicochemical barriers.

11. **Biosensor** according to the previous claims **characterized in that** the means of regulating the speed of circulation of the fluid are water-soluble barriers (10).

12. **Biosensor** according to the previous claims **characterized in that** the means of regulating the flow velocity of the fluid include alternative paths (12) for the flow of the fluid within the lateral flow strip.

13. **Biosensor** according to the previous claims **characterized in that** the means of regulating the speed of circulation of the fluid include the use of different membranes of porous material (13).

14. **Biosensor** according to the previous claims **characterized in that** the means of regulating the speed of circulation of the fluid include two or more methods of the following: water-soluble barriers (11) and/or alternative paths (12) for the flow of the fluid and/or use of different membranes of porous material (13).

15. **Biosensor** according to any of the above claims **characterized in that** the electrode (6) is an electrode made of a biologically and/or chemically modified material.

16. **Biosensor** according to the previous claims **characterized in that** the electrode (6) is made of carbon, graphene, gold or other screen-printed metal.

17. **Biosensor** according to claims 15 and 16 **characterized in that** the material is modified with a redox/conductor polymer or other redox/conductive molecules.

18. **Biosensor** according to any of the preceding claims **characterized in that** the lateral flow strip (14) contains the anti-target molecule of the specific analyte functionalized with a marker, such as HRP, gold nanoparticles, iron molecules, or any other indicator that generates a measurable signal.

19. **Biosensor** according to any of the previous claims **characterized by** comprising a sealing and insulation structure configured to prevent the evaporation of the liquid sample.

20. **Device for the autonomous realization of a measurement of an analyte in a liquid sample,** which includes at least:
• a lateral flow strip (14) comprised of one or more layers of porous material, with at least one placement zone of the liquid sample, and a capture zone in which the chemical and/or biological reactions of an analyte are carried out with some reagents present in the capture zone, where the lateral flow strip is hydrophilic and is configured to transport the fluid along the layer or layers of porous material;
• and an electrode (6) located in direct contact with the lateral flow strip, configured to interact with the target molecule of the measurement. **characterized in that** the biological recognition reaction occurs in the lateral flow strip (14) and/or on the surface of the electrode (6); **in that** the lateral flow strip (14) comprises means of regulating the velocity of circulation of the fluid along the lateral flow strip (14), configured to regulate the reaction time of each of the stages of chemical and/or biological reactions; and **in that** the device comprises an autonomous capillarity extraction element, where the capillarity effect of the extraction element is greater than the capillarity effect of the tissue from which the liquid sample is extracted.

21. **Device** according to the previous claim **characterized by** comprising a sealing and insulation structure configured to prevent the evaporation of the liquid sample.
